# EUROPÄISCHE PATENTANMELDUNG

(11) **EP 0 707 847 A1**
(43) Veröffentlichungstag der Anmeldung: **24.04.1996**
(21) Anmeldenummer: 95115899.7
(22) Anmeldetag: 09.10.1995
(51) Int. Cl.: A61K 9/127, A61K 31/19

(54) **Ketoprofen Liposomen**

(30) Priorität: 20.10.1994 IT MI942142
(71) Anmelder: BAYER AG, D-51368 Leverkusen (DE)
(72) Erfinder: Ciceri, Silvana, Dr., I-22100 Como (IT); Hamann, Hans-Jürgen, Dr., D-41539 Dormagen (DE); Hürner, Ingrid, D-40479 Düsseldorf (DE); Kurka, Peter, Dr., D-40723 Hilden (DE); Maasz, Joachim, Dr., Granger, IN 46530 (US)

(57) **Zusammenfassung**

Die vorliegende Erfindung betrifft ein Ketoprofen-Liposomen-Gel und ein Verfahren zu seiner Herstellung, das sich durch eine einfache Darstellungsweise durch spontane Bildung von Liposomen aus Phospholipiden und Ketoprofen auszeichnet.

## Beschreibung

Die vorliegende Erfindung betrifft ein Ketoprofen-Liposomen-Gel und ein Verfahren zu seiner Herstellung, das sich durch eine einfache Darstellungsweise durch spontane Bildung von Liposomen aus Phospholipiden und Ketoprofen auszeichnet.

Ketoprofen ist ein arzneilich wirksamer Stoff aus der Gruppe der Nicht-Steroidalen Anthrheumatika vom Typ der Arylpropionsäuren. Die Nicht-Steroidalen Antirheumatika werden sowohl innerlich als auch äußerlich zur Therapie von Entzündungen eingesetzt, wobei insbesondere die lipophilen Vertreter dieser Wirkstoffklasse für die topische Applikation geeignet sind. Ein Problem der Applikation von Arzneistoffen ist die schlechte Penetrierbarkeit der Haut für viele Arzneistoffe, so daß oftmals die notwendigen therapeutischen Spiegel im Gewebe nicht erreicht werden. Daher wird vielfach versucht durch Einschluß der Wirkstoffe in Liposomen die Penetrierbarkeit ins Gewebe durch das Stratum-Corneum zu verbessern.

Liposomen werden aus Phospholipiden gebildet, die sich in einer oder mehreren konzentrischen Schichten zu kugelförmigen flüssig-kristallinen Partikeln zusammenlagern. Die Größe von Liposomen variiert je nach Herstellungsart und Typus von ca. 80 nm bis 100 µm. Man Klassifiziert Liposomen durch ihre Größe und durch die Anzahl der Phospholipidschichten, wobei zwischen unilamellar und multilamellar, evtl. nur oligolamellar unterschieden wird.

Phospholipide sind amphiphile Substanzen, d.h. sie besitzen sowohl einen hydrophilen Molekülteil, meist einen mit einer quartären Ammoniumverbindung substituierten Phosphorsäureester und einen lipohilen Molekülteil der zumeist aus gesättigten und ungesättigten Fettsäuren besteht. Durch diese Eigenschaften besteht, wie bei allen Tensiden, die Tendenz sich in wäßrigen Systemen zu Assoziaten zusammenzulagern, um die Energie des Systems zu verringern. Phospholipide neigen dazu sich zu Doppelschichten zusammenzulagern, wobei die lipophilen Molekülteile zueinander orientiert sind und diese Doppelschichten durch wäßrige Kompartimente getrennt sind. Diese Doppelschichten können sich nun konzentrisch zu kugelförmigen Gebilden, Liposomen, anordnen. Liposomen bestehen entweder aus einer, oder mehreren Phospholipid-Doppelschichten, in die entweder hydrophile Substanzen in die wäßrigen Kompartimente, oder lipophile Substanzen in die Doppelschichten eingelagert werden.

Da die Phospholipide, aus denen Liposomen gebildet werden, zum Teil den physiologischen Membranlipiden und Lipidsubstanzen der Hornhaut entsprechen oder diesen sehr ähnlich sind, wird insbesondere topischen Liposomen eine größere Penetrationsfähigkeit durch die Haut zugesprochen. Dies trifft insbesondere für entzündete Haut zu, in die Liposomen gut eindringen können (H.E. Junginger et al, Cosm. Toiletr., 45-50, 1991).

Niosomen sind Vesikel von derselben Struktur wie die Liposomen, die jedoch aus nicht-ionischen Amphiphilen, wie mit Fettsäuren veretherten oder veresterten Polyoxyethylenen oder beispielsweise Fettsäure-Saccharosediestern gebildet werden.

Es wurden schon mannigfaltige Versuche gemacht Liposomenformulierungen auch von Nicht-Steroidalen Antirheumatika herzustellen, jedoch wird bei diesen Herstellungsverfahren immer mit einem hohen Energieaufwand gearbeitet, um eine ausreichende Partikelgröße zu erreichen oder es müssen organische Lösungsmittel oder Detergenzien eingesetzt werden, die im Anschluß aus der Formulierung wieder entfernt werden müssen. Ein Beispiel dafür bietet EP 0 249 561, worin Methoden und Zusammensetzung von Formulierungen in denen Nicht-Steroidale Antirheumatika in Liposomen eingeschlossen sind beschrieben werden. Das Patent beansprucht Liposomen zur oralen Anwendung und magensaftresistente Liposomen, das Herstellungsverfahren zur Darstellung dieser Liposomen entspricht jedoch den üblichen Methoden, d.h. die Phospholipide werden in einer organischen Phase gelöst, die im Anschluß wieder entfernt werden muß.

Übliche bekannte Herstellungsmethoden für Liposomen seien kurz aufgeführt:
1. Hydrationsmethode
   Ein Phospholipidgemisch wird in einem Glaskolben eingedampft, so daß sich ein dünner Lipidfilm an der Wandung ausbildet. Dieser Film wird dann mit einer Pufferlösung befeuchtet und geschüttelt, wobei sich spontan Liposomen ausbilden. Kritische Parameter bei diesem Prozeß sind die Lipidfilmdicke, das Volumen der Pufferlösung sowie die Dauer und Intensität der Bewegung. Die hierbei entstehenden, meist multilamellaren Liposomen können durch Ultraschall oder die sogenannte French Press noch weiter verkleinert werden.
2. Ultraschallmethode
   Die Phospholipide werden in Wasser dispergiert und danach durch mechanische Belastung, in diesem Falle Ultraschall zerkleinert. Alternativ können die Liposomen auch durch Druckbelastung in einem geeigneten Extruder (French Press) aus Dispersionen dargestellt werden.
3. Lösungsmittel-Injektionsmethode
   Die Phospholipide werden in einem geeigneten organischen Lösungsmittel gelöst (Ether, Methanol und Gemische) und diese Lösung in warmes Wasser eingespritzt, in dem der einzuschließende Stoff gelöst vorliegt. Nach Abzug des Lösungsmittels im Vakuum bilden sich zumeist unilamellar Vesikel aus.
4. Detergenzien-Methode
   Eine wäßrige Mischmizellösung aus Phospholipiden, einem Detergenz und der einzuschließenden Substanz wird hergestellt und das Detergenz im Anschluß durch Dialyse, Säulenchromatographie oder andere geeignete Verfahren abgetrennt.
5. Reverse Phase Evaporation Method
   Im Überschuß der organischen Phase wird mit einem Puffer, den Phospholipiden und den einzuschließenden Substanzen eine Wasser-in-Öl-Emulsion hergestellt und dann die organische Phase im Vakuum abgedampft. Am Ende des Verdampfungsvorgangs kommt es zu einer Phasenumkehr und es bildet sich eine Suspension von großen unilamellaren Liposomen.

Diese Methoden sind in ihrer technischen Durchführung alle, entweder bei mechanischer Darstellung der Liposomen mit einem hohen Energieaufwand, oder bei den Methoden mit Lösungsmitteln oder Detergenzien mit einem hohen Reinigungsaufwand des Produktes, verbunden. Es besteht daher der Wunsch nach einer einfachen Darstellung solcher Systeme.

Überraschend wurde gefunden, daß sich Ketoprofen-Liposomen äußerst einfach durch Mischen von Ketoprofen mit Phospholipiden bei pH-Werten über 6, vorzugsweise 6 bis 8, und anschließende pH-Senkung auf Werte unter 6, vorzugsweise 4 bis 6, darstellen lassen. Das Natriumsalz des Ketoprofens besitzt durch die deprotonierte Carboxylgruppe amphiphilen Charakter und lagert sich mit den Phospholipiden zusammen und es entstehen Mischmizellen aus dem eingesetzten Phospholipid und dem Ketoprofen-Salz. In diese Mischmizellen ist die noch vorliegende freie Säure, das Ketoprofen, inkorporiert. Bei Verdünnung dieser alkalischen Mischmizellösung mit einer geeigneten Pufferlösung senkt sich der pH-Wert der Lösung auf einen Wert unter 6, der Anteil des deprotonierten Ketoprofens verringert sich. Dadurch wird die Mischmizellmembran destabilisiert und es kommt zur spontanen Bildung von Liposomen. Entscheidend ist dabei insbesondere, daß nicht wie bei vielen anderen Herstellungsverfahren für Liposomen mit organischen Lösungsmitteln (reverse phase evaporation method) oder Detergenzien, die aus der Formulierung entfernt werden müssen (Detergent removal method) oder energieaufwendigen Zerkleinerungsmethoden (Hydration method, sonication method, French press etc.) gearbeitet werden muß. Die Formulierung enthält sowohl das entsprechende Salz des Ketoprofens, als auch die freie Säure, das Ketoprofen. Die Partikelgrößen liegen im Bereich von 80 bis 200 nm.

Geeignete Phospholipide sind die natürlichen Phospholipide, Sphingolipide, Glycosphingolipide, sowie synthetische Phospholipide wie Dipalmitoylphosphatidylcholin-, -serin, -ethanolamin-glycerol oder die entsprechenden Ölsäureester dieser Verbindungen.

Besonders geeignet sind natürliche und synthetische Phospholipide, die der allgemeinen Formel (I) 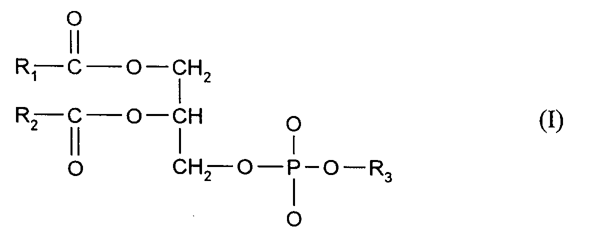
entsprechen sowie deren Mischungen, wobei R₁ und R₂ Alkylreste und/oder einfach- bis vierfach ungesättigte Alkenylgruppen mit 10 bis 23, vorzugsweise 13 bis 21 C-Einheiten bedeuten und R₃ aus der folgenden Gruppe stammt:
-OH,
-CH₂CH₂N⁺(CH₃)₃,
-CH₂CH₂NH₃⁺,
-CH₂CHNH₃⁺COO-,
-CH₂CHOHCH₂OH,
-HC₆H₅(OH)₅.

Geeignete nicht-ionische Amphiphile sind vor allem mit Fettsäuren veresterte oder veretherte Polyoxyethylene und Saccharosediester, die zur Bildung von Niosomen geeignet sind.

Bevorzugte nicht-ionische Amphiphile sind Verbindungen der Formeln

R₄-O-(CH₂-CH₂-O)ₙH (II)

und 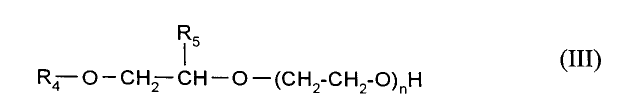
wobei R₄ und R₅ gleich oder verschieden sind und jeweils für Alkyl oder Alkenyl mit 12 bis 16 C-Atomen stehen und
n für eine Zahl von 3 bis 25 steht.

Geeignete Gelbildner sind die dem Fachmann bekannten Hydrogelbildner, wie derivatisierte Cellulosen, Polyvinylpyrrolidone, Polyacrylate und andere synthetische Hydrokolloide, sowie natürliche Gelbildner wie Agar, Gummen, Glykane, Alginate, Gelatine und andere Polysaccharid- und Protein-Hydrokolloide und Blockcopolymere des Polyoxyethylens und Polyoxypropylens.

Als übliche Hilfsstoffe seien insbesondere genannt Konservierungsmittel, Antioxidantien, Farbstoffe und andere Stoffe die zur mikrobiellen und chemischen Stabilisierung der Formulierung dienen.

Erfindungsgemäße Ketoprofen-Liposomen-Gele werden z.B. durch Einarbeiten von Ketoprofen-Phospholipid-Mischmizellen in bestimmte Hydrogele hergestellt. Dazu wird zunächst Ketoprofen in 1NNaOH-Lösung gelöst und in diesem Medium durch Einarbeiten eines Phospholipids, insbesondere der Formel (I) eine Mischmizelldispersion hergestellt. Geeignete Phospholipide sind natürliche und synthetische Phospholipide, die ungesättigte und gesättigte Fettsäuren enthalten können (Beispiele: Phospholipon®90, Lipoid®E 80, Lipoid® E 100, Lipoid® S 100, Lipoid® E PC, Epikuron® 200 SH).

### Ausführungsbeispiele

### Beispiel 1

### Herstellung der Mischmizellösung

| | |
|---|---|
| Ketoprofen | 18,62 g |
| Phospholipon®90 | 17,11 g |
| 1N-NaOH-Lösung | 100,0 g |

Ketoprofen wird in der Natronlauge gelöst. Anschließend wird die Lösung auf 90°C erhitzt, und das Phospholipid darin über 90 Minuten dispergiert. Es entsteht eine klare gelbe Mischmizelldispersion mit einem pH-Wert über 6.

Die Mischmizellösung wird in folgendes Hydrogel eingearbeitet:

| | |
|---|---|
| Lutrol® F 127 | 18,00 % |
| Pufferlösung pH 5 | 63,58 % |
| Cremophor® RH 40 | 5,00 % |
| Mischmizelldispersion | 13,42 % |

Durch das Verdünnen der Mischmizellösung mit dem Hydrogel bildet sich ein stabiles Ketoprofen-Liposomen-Gel. Die Konzentration von Ketoprofen im Gel beträgt 2,5 %.

### Beispiel 2

Die Mischmizellösung aus Beispiel 1 wird in folgende Hydrogel eingearbeitet:

| | |
|---|---|
| Polyacrylsäure | 1,00 % |
| Pufferlösung pH 5 | 80,33 % |
| Cremophor® RH 40 | 5,00 % |
| Mischmizelldispersion | 13,42 % |

Durch das Verdünnen der Mischmizellösung mit dem Hydrogel bildet sich ein stabiles Ketoprofen-Liposomen-Gel. Die Konzentration von Ketoprofen im Gel beträgt 2,5 %.

## Patentansprüche

1. Verfahren zur Herstellung von Ketoprofen-Liposomen, dadurch gekennzeichnet, daß eine Mischung von Ketoprofen und Phospholipiden oder nicht-ionischen Amphiphilen in eine Lösung mit einem pH-Wert von 6 bis 8 gegeben wird und in dieser Lösung anschließend der pH-Wert auf Werte unter 6 abgesenkt wird, wobei es zur spontanen Bildung von Liposomen kommt, welche gegebenenfalls mit üblichen Hilfsstoffen in halbfeste und flüssige Zubereitungen überführt werden.

2. Verfahren zur Herstellung von Ketoprofen-Liposomen gemäß Anspruch 1, dadurch gekennzeichnet, daß man als nicht-ionische Amphiphile Verbindungen der allgemeinen Formeln (II) bzw. (III) einsetzt
R₄-O-(CH₂-CH₂-O)ₙH (II)
und 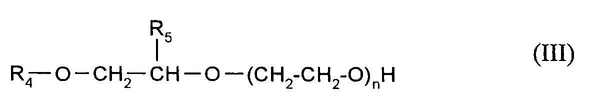 wobei R₄ und R₅ gleich oder verschieden sind und jeweils für Alkyl oder Alkenyl mit 12 bis 16 C-Atomen stehen und
n für eine Zahl von 3 bis 25 steht.

3. Arzneizubereitungen enthaltend Ketoprofen-Liposomen die aus Phospholipiden der allgemeinen Formel (I) 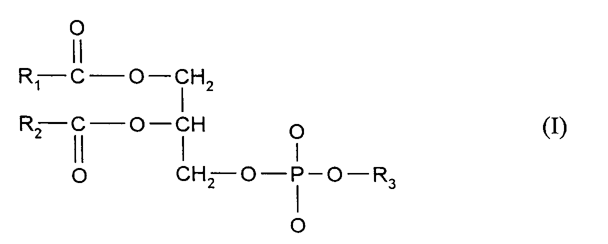 wobei R₁ und R₂ Alkylreste und/oder einfach- bis vierfach ungesättigte Alkenylgruppen mit 10 bis 23, vorzugsweise 13 bis 21 C-Einheiten bedeuten und R₃ aus der folgenden Gruppe stammt:
-OH,
-CH₂CH₂N⁺(CH₃)₃,
-CH₂CH₂NH₃⁺,
-CH₂CHNH₃⁺COO-,
-CH₂CHOHCH₂OH,
-HC₆H₅(OH)₅,
sowie deren Mischungen und Ketoprofen und/oder Ketoprofen-Natriummischmizellen und gegebenenfalls weiteren üblichen Hilfsstoffen aufgebaut sind.

4. Arzneizubereitungen gemäß Anspruch 3, dadurch gekennzeichnet, daß die Ketoprofen-Liposomen in Hydrogele eingearbeitet sind.
